(19)

Europäisches Patentamt
European Patent Office
Office européen des brevets

(11) **EP 4 174 760 A1**

(12) **EUROPEAN PATENT APPLICATION**
published in accordance with Art. 153(4) EPC

(43) Date of publication:
**03.05.2023 Bulletin 2023/18**

(21) Application number: **20943267.3**

(22) Date of filing: **30.11.2020**

(51) International Patent Classification (IPC):
**G06T 7/00** (2017.01)          **G06N 20/00** (2019.01)

(52) Cooperative Patent Classification (CPC):
**G06N 20/00; G06T 7/00**

(86) International application number:
**PCT/CN2020/132796**

(87) International publication number:
**WO 2022/000976 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2020   CN 202010606964**
              **29.06.2020   CN 202010606963**

(71) Applicant: **Suzhou Rainmed Medical Technology Co., Ltd.**
**Suzhou, Jiangsu 215000 (CN)**

(72) Inventors:
• **FENG, Liang**
  **Suzhou, Jiangsu 215000 (CN)**
• **LIU, Guangzhi**
  **Suzhou, Jiangsu 215000 (CN)**
• **WANG, Zhiyuan**
  **Suzhou, Jiangsu 215000 (CN)**

(74) Representative: **Boult Wade Tennant LLP**
**Salisbury Square House**
**8 Salisbury Square**
**London EC4Y 8AP (GB)**

(54) **AORTA OBTAINING METHOD BASED ON DEEP LEARNING, AND STORAGE MEDIUM**

(57)    The present application provides a method for acquiring aorta based on deep learning and a storage medium, comprising: acquiring a database of slices of an aortic layer and a database of slices of a non-aortic layer; performing deep learning on the database of slices of the aortic layer and the database of slices of the non-aortic layer, to obtain a deep learning model; acquiring CT sequence images to be processed or three-dimensional data of CT sequence images to be processed; extracting feature data from the CT sequence images to be processed or the three-dimensional data of the CT sequence images to be processed; acquiring an image of the aorta from the CT sequence images based on the deep learning model and the feature data. The present application acquires the deep learning model based on the feature data and the database, and acquires the image of aorta by the deep learning model, which has the advantages of good extraction effect, high robustness, and accurate calculation results, and has high promotion value in clinical practice.

FIG.1

**EP 4 174 760 A1**

## Description

## TECHNICAL FIELD

The present invention refers to the technical field of coronary medicine, and in particular to methods for acquiring aorta based on deep learning and storage media.

## BACKGROUND

Cardiovascular diseases are leading causes of death in the industrialized world. The major forms of cardiovascular diseases are caused by chronic accumulation of fatty material in the inner tissue layers of the arteries supplying the heart, brain, kidneys and lower extremities. Progressive coronary artery diseases restrict blood flow to the heart. Due to the lack of accurate information provided through current non-invasive tests, invasive catheterization procedures are required by many patients to evaluate coronary blood flow. Thus, a need exists for non-invasive methods for quantifying blood flow in human coronary arteries to evaluate the functional significance of possible coronary artery diseases. Reliable evaluation of arterial volume will therefore be important for disposition planning to address patient needs. Recent studies have demonstrated that hemodynamic characteristics, such as flow reserve fraction (FFR), are important indicators for determining the optimal disposition for patients with arterial disease. Routine evaluation of FFR uses invasive catheterization to directly measure blood flow characteristics, such as pressure and flow rate. However, these invasive measurement techniques carry risks to the patient and can result in significant costs to the health care system.

Computed tomography arteriography is a computed tomography technique used to visualize the arterial blood vessels. For this purpose, a beam of X-rays is passed from an radiation source through the area of interest in the patient's body to obtain a projection image.

The use of empirical values to acquire images of an aorta in prior art suffers from many human factors, poor consistency, and slow extraction speed.

## SUMMARY

The present invention provides a method for acquiring aorta based on deep learning and a storage medium, to solve the problems of the prior art of using empirical values to acquire images of aorta with many human factors, poor consistency and slow extraction speed.

To achieve the above, in a first aspect, the present application provides a method for acquiring aorta based on deep learning, comprises:

acquiring a database of slices of an aorticlayer and a database of slices of a non-aortic layer;

performing deep learning on the database of slices of the aortic layer and the database of slices of the non-aortic layer, to obtain a deep learning model;

acquiring CT sequence images to be processed or three-dimensional data of CT sequence images to be processed;

extracting feature data from the CT sequence images to be processed or the three-dimensional data of the CT sequence images to be processed;

acquiring an image of the aorta from the CT sequence images based on the deep learning model and the feature data.

Optionally, in the above method for acquiring aorta based on deep learning, the manner for acquiring a database of slices of an aortic layer and a database of slices of a non-aortic layer comprises:

removing new images of the lung, descending aorta, spine and ribs from CTsequence images;

slicing starting from a top layer of the new images to obtain a group of two-dimensional images;

binarizing the group of two-dimensional images to obtain a group of binarized images;

generating an image of the aorta based on each group of binarized images;

generating slice data of the aortic layer based on the image of the aorta, with the remaining slice data being as slice data of the non-aortic layer, and obtaining a database of slices of the aortic layer and a database of slices of the non-aortic layer.

Optionally, in the above method for acquiring aorta based on deep learning, the manner for binarizing the group of two-dimensional images to obtain a group of binarized images comprises:

setting a coronary tree grayscale threshold $Q_{coronary\ 1}$; based on

$$\left\{ \begin{array}{l} 0 \leq Q_m < Q_{coronary\ 1}, P(m) = 0 \\ Q_{coronary\ 1} \leq Q_m \leq 255, P(m) = 1 \end{array} \right\}_{bi-}$$

narizing the slices of each layer of the new images with the lung, descending aorta, spine, and ribs removed to remove impurity points in the new images and obtain a group of binarized images;

where m is a positive integer, $Q_m$ denotes the grayscale value corresponding to the m-th pixel point PO, and P(m) denotes the pixel value corresponding to the m-th pixel point PO.

Optionally, in the above method for acquiring aorta based on deep learning, the manner for binarizing the group of two-dimensional images to obtain a group of binarized images comprises:

creating a search engine list for each layer of slices in the group of binarized images;

searching for circles in each layer of slices, comparing the number of pixel points in the search engine list of each layer and the radii of the circles, and finding an eligible circle center point;

searching for a circle center point of the next layer of slice, if no eligible circle center point can be found.

Optionally, in the above method for acquiring aorta based on deep learning, the manner for removing new images of the lung, descending aorta, spine and ribs from CT sequence images comprises:

acquiring a three-dimensional database of CT sequence images;

plotting a grayscale histogram of each group of the CT sequence images;

along a direction of the end point M to the original point O of the grayscale histogram, acquiring a volume of each grayscale value region from point M to point M-1, from point M to point M-2, until from point M to point O; acquiring a volume ratio V of the volume of each grayscale value region to a volume of the total region from point M to point O;

setting a lung grayscale threshold $Q_{lung}$ based on medical knowledge and CT imaging principle; if a grayscale value in the grayscale histogram being less than $Q_{lung}$, removing an image corresponding to the grayscale value to obtain a first image with the lung tissue removed;

acquiring a gravity center of heart and a gravity center of spine corresponding to each group of CT sequence images based on the first image;

acquiring an image of descending aorta for each group of CT sequence images based on the gravity center of heart and the gravity center of spine;

removing the lung, descending aorta, spine, and ribs from the CT sequence images to acquire new images.

Optionally, in the above method for acquiring aorta based on deep learning, the manner for acquiring a gravity center of heart and a gravity center of spine corresponding to each group of CT sequence images based on the first image comprises:

if V = b, picking a start point corresponding to the grayscale value region, projecting the start point onto the CT three-dimensional image, acquiring a three-dimensional image of a heart region, and picking a physical gravity center of the three-dimensional image of the heart region, which is the gravity center of heart $P_2$; wherein b denotes a constant, $0.2 < b < 1$;

if V = a, picking a start point corresponding to the grayscale value region, projecting the start point onto the CT three-dimensional image, acquiring a three-dimensional image of a bone region, and picking a physical gravity center of the three-dimensional image of the bone region, which is the gravity center of spine $P_1$; wherein a denotes a constant, $0 < a < 0.2$.

Optionally, in the above method for acquiring aorta based on deep learning, the manner for acquiring an image of descending aorta for each group of CT sequence images based on the gravity center of heart and the gravity center of spine comprises:

setting a lung grayscale threshold $Q_{lung}$ based on medical knowledge and CT imaging principle;

if a grayscale value in the grayscale histogram being less than $Q_{lung}$, removing an image corresponding to the grayscale value to obtain a first image with the lung tissue removed;

projecting the gravity center of heart $P_2$ onto the first image to obtain a circle center of the heart $O_1$;

setting a grayscale threshold for the descending aorta $Q_{descending}$, and binarizing the first image;

acquiring a circle corresponding to the descending aorta based on a distance from the descending aorta to the circle center of the heart $O_1$ and a distance from the spine to the circle center of the heart $O_1$;

acquiring an image of the aorta from the CT sequence image.

Optionally, in the above method for acquiring aorta based on deep learning, the manner for setting a grayscale threshold for the descending aorta $Q_{descending}$ and binarizing the first image comprises:

acquiring one or more pixel point PO within the first image with a grayscale value greater than the grayscale threshold for the descending aorta $Q_{descending}$, and calculating an average grayscale value $\overline{Q}_1$ of the

one or more pixel point PO;

layered slicing the first image starting from its bottom layer to obtain a first group of two-dimensional sliced images;

based on

$$\left\{ \begin{array}{l} Q_k < Q_{descending}, P(k) = 0 \\ Q_{descending} \leq Q_k \leq 2\overline{Q}_1, P(k) = 1 \\ Q_k > 2\overline{Q}_1, P(k) = 0 \end{array} \right\}$$

, binarizing the first image, removing impurity points in the first image to obtain a binarized image, wherein k is a positive integer, $Q_k$ denotes the grayscale value corresponding to the k-th pixel point PO, and P(k) denotes the pixel value corresponding to the k-th pixel point PO.

Optionally, in the above method for acquiring aorta based on deep learning, the manner for acquiring a circle corresponding to the descending aorta based on a distance from the descending aorta to the circle center of the heart $O_1$ and a distance from the spine to the circle center of the heart $O_1$ comprises:

setting an radius threshold of the circle formed from the descending aorta to an edge of the heart to $r_{threshold}$;

acquiring an approximate region of the spine and an approximate region of the descending aorta based on the distance between the descending aorta and the heart being less than the distance between the spine and the heart;

removing one or more error pixel points based on the approximate region of the descending aorta, and obtaining an image of the descending aorta, i.e., a circle corresponding to the descending aorta.

Optionally, in the above method for acquiring aorta based on deep learning, the manner for acquiring an approximate region of the spine and an approximate region of the descending aorta based on the distance between the descending aorta and the heart being less than the distance between the spine and the heartcomprises that:

if a circle obtained by the Hoff detection algorithm meets the condition that its radius $r > r_{threshold}$, then this circle is the circle corresponding to the spine and is the approximate region of the spine, and the center and radius need not to be recorded;

if a circle obtained by the Hoff detection algorithm meets the condition that its radius $r \leq r_{threshold}$, then this circle may be the circle corresponding to the descending aorta and is the approximate region of the descending aorta, and the center and radius need to be recorded.

Optionally, in the above method for acquiring aorta based on deep learning, the manner for removing one or more error pixel points based on the approximate region of the descending aorta, and obtaining an image of the descending aorta, i.e., a circle corresponding to the descending aorta, comprises:

screening the centers and radii of the circles within the approximate region of the descending aorta, removing the circles with centers of large deviations between adjacent slices, i.e., removing the one or more error pixel points, and forming a list of seed points of the descending aorta to obtain an image of the descending aorta.

Optionally, in the above method for acquiring aorta based on deep learning, the manner for extracting feature data from the CT sequence images to be processed or the three-dimensional data of the CT sequence images to be processed comprises:

plotting a grayscale histogram of the CT sequence images to be processed;

acquiring an average grayscale value $Q_1$ of one or more pixel points with a grayscale value greater than $Q_{descending}$;

layered slicing the CT sequence images to obtain a group of two-dimensional sliced images;

binarizing all the images in the group of two-dimensional sliced images based on the average grayscale value$\overline{Q}_1$, to obtain a plurality of binarized images;

acquiring a connected domain of each binarized image successively starting from the top layer, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer $C_1$ corresponding to the connected domain, and an area $M_k$ of all pixels whose pixel points are greater than 0 in a layer pixel and whose pixel points are equal to 0 in the previous layer pixel and a filtered area $H_k$, wherein k denotes the k-th layer of slice, $k \geq 1$;

making the proposed circle center $C_k$, the distance $C_k$-$C_{(k-1)}$, the areas $S_k$, $M_k$, $H_k$, the proposed circle radius $R_k$, and the distance $L_{k-(k-1)}$ between two adjacent layers corresponding to the connected domainas feature data.

Optionally, in the above method for acquiring aorta based on deep learning, the manner for acquiring an image of the aorta from the CT sequence images based on the deep learning model and the feature data comprises:

analyzing the feature data based on the deep learning model to obtain descending aortic data;

expanding the descendingaortic data;

multiplying the expanded descendingaortic data with original CTsequence image data, and calculating a gradient of each pixel point to obtain gradient data;

extracting a gradient edge based on the gradient data;

subtracting the gradient edge from the expanded aortic data;

generating a list of seed points based on a proposed circle center;

extracting a connected domain based on the list of seed points, to obtain an image of the aorta.

Optionally, in the above method for acquiring aorta based on deep learning, the manner for acquiring a connected domain of each binarized image successively starting from the top layer, as well as a proposed circle center $C_k$, an area $S_k$, a proposed circle radius $R_k$, and a distance $L_{k-(k-1)}$ between two adjacent layers, wherein k denotes the k-th layer of slice, comprises:

detecting 3 layers of slice successively starting from the top layer by using the Hoff detection algorithm, and obtaining 1 circle center and 1 radius from each layer of slice, forming 3 circles respectively;

removing points with larger deviations from 3 circle centers to obtain a seed point $P_1$ of the descending aorta;

acquiring a connected domain $A_1$ of the layer where the seed point $P_1$ is located;

acquiring a gravity center of the connected domain $A_1$ as the proposed circle center $C_1$, and acquiring the area $S_1$ of the connected domain $A_1$ and the proposed circle radius $R_1$;

acquiring a connected domain $A_2$ of the layer where the seed point $P_1$ is located, by using the $C_1$ as a seed point;

expanding the connected domain $A_1$ to obtain an expanded region $D_1$, removing a portion overlapping with the expanded region $D_1$ from the connected domain $A_2$ to obtain a connected domain $A_2$';

setting a volume threshold $V_{threshold}$ for the connected domain, if a volume $V_2$ of the connected domain $A_2$' being less than $V_{threshold}$, removing a point that is too far from the circle center $C_1$ of the previous

layer, acquiring the filtered area $H_k$, making the gravity center of the connected domain $A_2$' as a proposed circle center $C_2$, acquiring an area $S_2$ of the connected domain $A_2$ and a proposed circle radius $R_2$;

repeating the method of the connected domain $A_2$, acquiring a connected domain of each binarized image successively, as well as a proposed circle center $C_k$, an area $S_k$, a proposed circle radius $R_k$, and a distance $C_k-C_{(k-1)}$ between the circle centers of two adjacent layers, a distance $C_k-C_1$ from the circle center $C_k$ of each layer of slice to the circle center of the top layer $C_1$ corresponding to the connected domain.

In a second aspect, the present application provides a computer storage medium, the above method for acquiring aorta based on deep learning is implemented when a computer program is executed by a processor.

The beneficial effects resulting from the solutions provided by embodiments of the present application include at least that:
the present application provides a method for acquiring aorta based on deep learning, acquires the deep learning model based on the feature data and the database, and acquires the image of aorta by the deep learning model, which has the advantages of good extraction effect, high robustness, and accurate calculation results, and has high promotion value in clinical practice.

**BRIEF DESCRIPTION OF DRAWINGS**

The drawings illustrated herein are used to provide a further understanding of the present invention, form a part of the present invention, and the schematic embodiments of the invention and their descriptions are used to explain the present invention and do not constitute an undue limitation of the present invention. Wherein:

FIG. 1 is a flow chart of the method for acquiring aorta based on deep learning of the present application;

FIG. 2 is a flowchart of S100 of the present application;

FIG. 3 is a flowchart of S110 of the present application;

FIG. 4 is a flowchart of S116 of the present application;

FIG. 5 is a flowchart of S1164 of the present application;

FIG. 6 is a flowchart of S1165 of the present application;

FIG. 7 is a flowchart of S140 of the present application;

FIG. 8 is a flowchart of S400 of the present application;

FIG. 9 is a flowchart of S450 of the present application;

FIG. 10 is a flowchart of S500 of the present application.

**DETAILED DESCRIPTION**

In order to make the purpose, technical solutions and advantages of the present invention clearer, the following will be a clear and complete description of the technical solutions of the present invention in conjunction with specific embodiments of the present invention and the corresponding drawings. Obviously, the described embodiments are only a part of the embodiments of the present invention, and not all of them. Based on the embodiments in the present invention, all other embodiments obtained by a person of ordinary skill in the art without making creative labor fall in the protection scope of the present invention.

A number of embodiments of the present invention will be disclosed in the following figures, and for the sake of clarity, many of the practical details will be described together in the following description. It should be understood, however, that these practical details should not be used to limit the present invention. That is, in some embodiments of the present invention, these practical details are not necessary. In addition, for the sake of simplicity, some of the commonly known structures and components will be illustrated in the drawings in a simple schematic manner.

The use of empirical values to acquire images of aorta in prior art suffers from many human factors, poor consistency, and slow extraction speed.

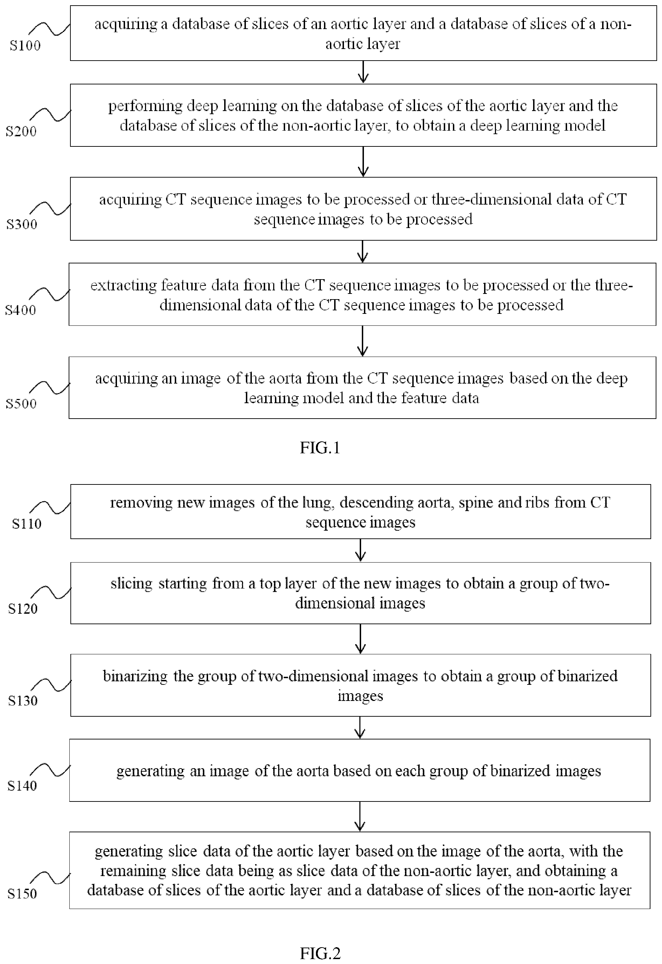

To solve the above problems, the present application provides a method for acquiring aorta based on deep learning, as shown in FIG. 1, comprising:

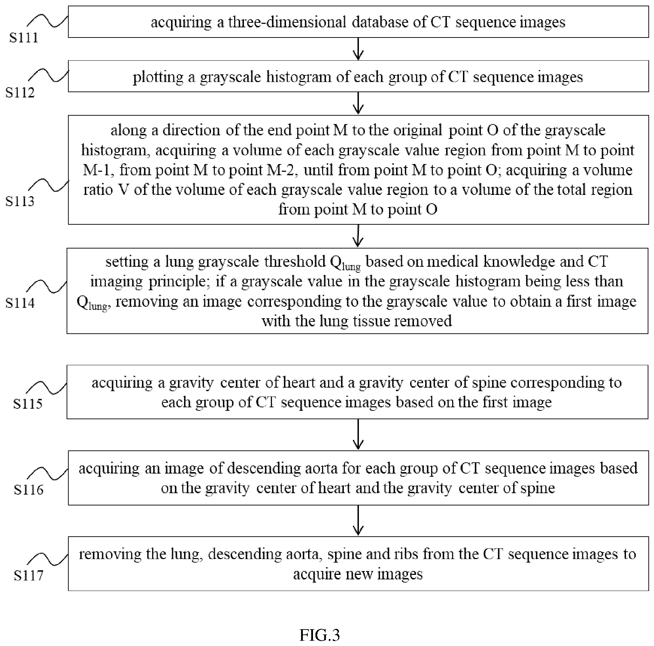

> S100, as shown in FIG. 2, acquiring a database of slices of an aorticlayer and a database of slices of a non-aortic layer, comprising:
> S110, as shown in FIG. 3, removing new images of the lung, descending aorta, spine, and ribs from CT sequence images, comprising:
>
>> Sill, acquiring a three-dimensional database of CT sequence images;
>>
>> S112, plotting a grayscale histogram of each group of CT sequence images;
>>
>> S113, along a direction of the end point M to the original point O of the grayscale histogram, acquiring a volume of each grayscale value region from point M to point M-1, from point M to point M-2, until from point M to point O; acquiring a volume ratio V of the volume of each grayscale value region to a volume of the total region from point M to point O;

S114, setting a lung grayscale threshold $Q_{lung}$ based on medical knowledge and CT imaging principle; if a grayscale value in the grayscale histogram being less than $Q_{lung}$, removing an image corresponding to the grayscale value to obtain a first image with the lung tissue removed;

S115, acquiring a gravity center of heart and a gravity center of spine corresponding to each group of CT sequence images based on the first image, comprising:

> if V = b, picking a start point corresponding to the grayscale value region, projecting the start point onto a CT three-dimensional image, acquiring a three-dimensional image of a heart region, and picking a physical gravity center of the three-dimensional image of the heart region, which is the gravity center of the heart $P_2$; wherein b denotes a constant, $0.2 < b < 1$;
>
> if V = a, picking a start point corresponding to the grayscale value region, projecting the start point onto a CT three-dimensional image, acquiring a three-dimensional image of a bone region, and picking a physical gravity center of the three-dimensional image of the bone region, which is the gravity center of spine $P_1$; wherein a denotes a constant, $0 < a < 0.2$;

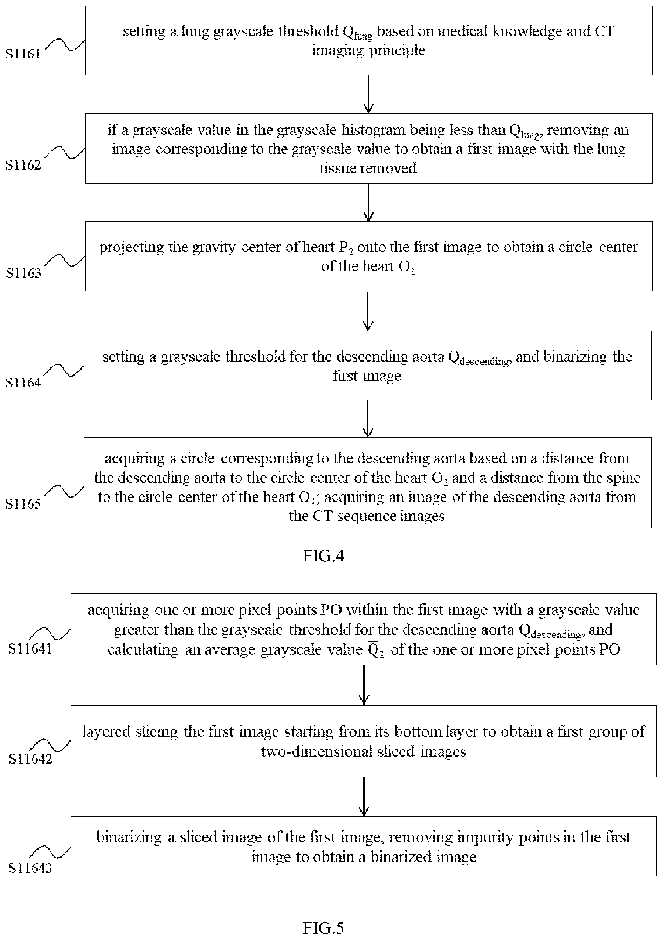

S116, as shown in FIG. 4, acquiring an image of a descending aorta for each group of CT sequence images based on the gravity center of heart and the gravity center of spine, comprising:

> S1161, setting a lung grayscale threshold $Q_{lung}$ based on medical knowledge and CT imaging principle;
>
> S1162, if a grayscale value in the grayscale histogram being less than $Q_{lung}$, removing an image corresponding to the grayscale value to obtain a first image with the lung tissue removed;
>
> S1163, projecting the gravity center of heart $P_2$ onto the first image to obtain a circle center of the heart $O_1$;

S1164, as shown in FIG. 5, setting a grayscale threshold for the descending aorta $Q_{descending}$, and binarizing the first image, comprising:

S11641, acquiring one or more pixel points PO within the first image with a grayscale value greater than the grayscale threshold for the descending aorta $Q_{descending}$, and calculating an average grayscale value $\overline{Q}_1$ of the one or more pixel points PO;

S11642, layered slicing the first image starting from its bottom layer to obtain a first group of two-dimensional sliced images;

S11643, based on

$$\begin{cases} Q_k < Q_{descending} \ , P(k) = 0 \\ Q_{descending} \ \le Q_k \le 2\overline{Q}_1, \ P(k) = 1 \\ Q_k > 2\overline{Q}_1, P(k) = 0 \end{cases},$$

binarizing a sliced image of the first image, removing impurity points in the first image to obtain a binarized image, where k is a positive integer, $Q_k$ denotes the grayscale value corresponding to the k-th pixel point PO, and P(k) denotes the pixel value corresponding to the k-th pixel point PO.

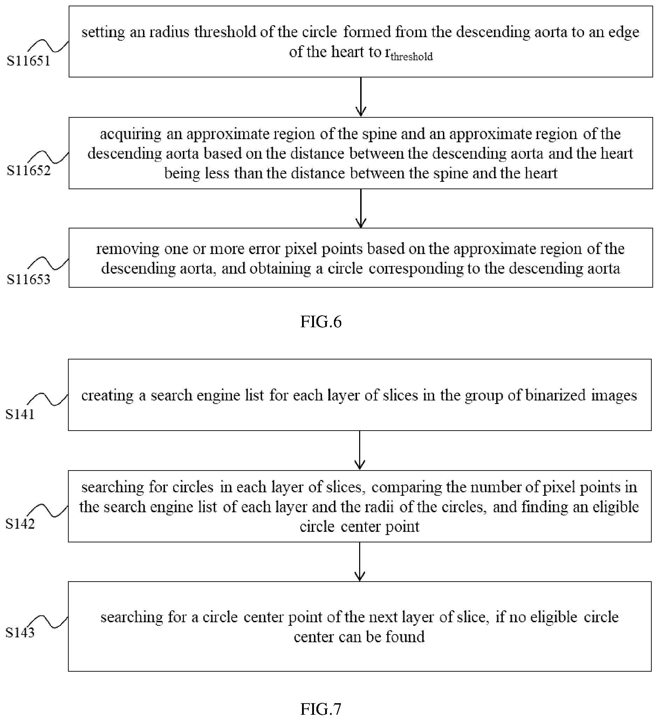

S1165, as shown in FIG. 6, acquiring a circle corresponding to the descending aorta based on a distance from the descending aorta to the circle center of the heart $O_1$ and a distance from the spine to the circle center of the heart $O_1$, comprising:

S11651, setting an radius threshold of the circle formed from the descending aorta to an edge of the heart to $r_{threshold}$;

S11652, acquiring an approximate region of the spine and an approximate region of the descending aorta based on the distance between the descending aorta and the heart being less than the distance between the spine and the heart, comprising:

if a circle obtained by the Hoff detection algorithm meets the condition that its radius $r > r_{threshold}$, then this circle is the circle corresponding to the spine and is the approximate region of the spine, and the center and radius need not to be recorded;

if a circle obtained by the Hoff detection algorithm meets the condition that its radius $r \le r_{threshold}$, then this circle may be the circle corresponding to the descending aorta and is the approximate region of the descending aorta, and the center and radius need to be recorded.

S11653, removing one or more error pixel points based on the approximate region of the descending aorta, and obtaining a circle corresponding to the descending aorta, comprising:

screening the centers and radii of the circles within the approximate region of the descending aorta, removing the circles with centers of large deviations between adjacent slices, i.e., removing the one or more error pixel points, and forming a list of seed points of the descending aorta to obtain an image of the descending aorta; acquiring the image of the descending aorta from a CT sequence image.

S117, removing the lung, descending aorta, spine, and ribs from the CT sequence images to acquire new images.

S120, slicing starting from a top layer of the new images to obtain a group of two-dimensional images;

S130, binarizing the group of two- dimensional images to obtain a group of binarized images, comprising:

setting a coronary tree grayscale threshold $Q_{coronary\ 1}$; based on

$$\begin{cases} 0 \le Q_m < Q_{coronary\ 1}, P(m) = 0 \\ Q_{coronary\ 1} \le Q_m \le 255, P(m) = 1 \end{cases},$$

binarizing the slices of each layer of the new images with the lung, descending aorta, spine, and ribs removed to remove impurity points in the new images and obtain a group of binarized images; where m is a positive integer, $Q_m$ denotes the grayscale value corresponding to the m-th pixel point PO, and P(m) denotes the pixel value corresponding to the m-th pixel point PO.

S140, as shown in FIG. 7, generating an image of the aorta based on each group of binarized images, comprising:

S141, creating a search engine list for each layer of slices in the group of binarized images;

5142, searching for circles in each layer of slices, comparing the number of pixel points in the search engine list of each layer and the radii of the circles, and finding an eligible circle center point;

S143, searching for a circle center point of the next layer of slice, if no eligible circle center point can be found.

S150, generating slice data of the aortic layer based on the image of the aorta, with the remaining slice data being as slice data of the non-aortic layer, and obtaining a database of slices of the aortic layer and a database of slices of the non-aortic layer.

By first screening out the gravity center of heart and the gravity center of spine, locating the position of the heart and the spine, and then acquiring the image of the descending aorta based on the position of the heart and the spine, computation burden is reduced, with simple algorithms, easy operation, fast computing speed, scientific design and accurate image processing.

S200, performing deep learning on the database of slices of the aortic layer and the database of slices of the non-aortic layer, to obtain a deep learning model;

S300, acquiring CT sequence images to be processed or three-dimensional data of CT sequence images to be processed;

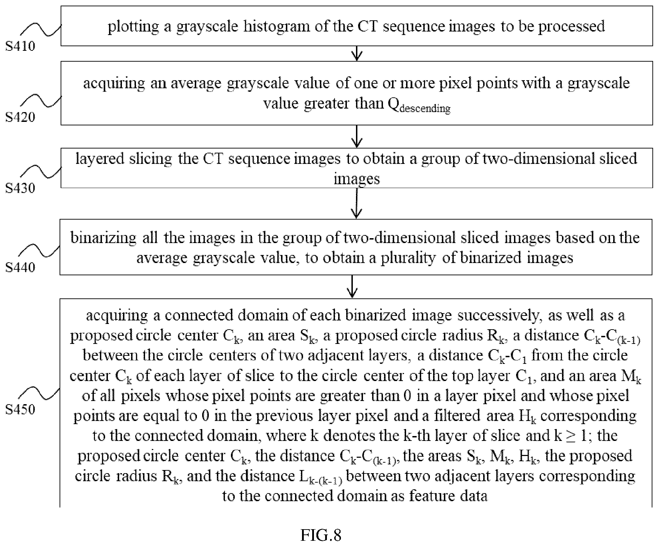

S400, as shown in FIG. 8, extracting feature data from the CT sequence images to be processed or the three-dimensional data of the CT sequence images to be processed, comprising:

S410, plotting a grayscale histogram of the CT sequence images to be processed;

S420, acquiring an average grayscale value $\overline{Q}_1$ of one or more pixel points with a grayscale value greater than $Q_{descending}$;

S430, layered slicing the CT sequence images to obtain a group of two-dimensional sliced images;

S440, binarizing all the images in the group of two-dimensional sliced images based on the average grayscale value $\overline{Q}_1$, to obtain a plurality of binarized images;

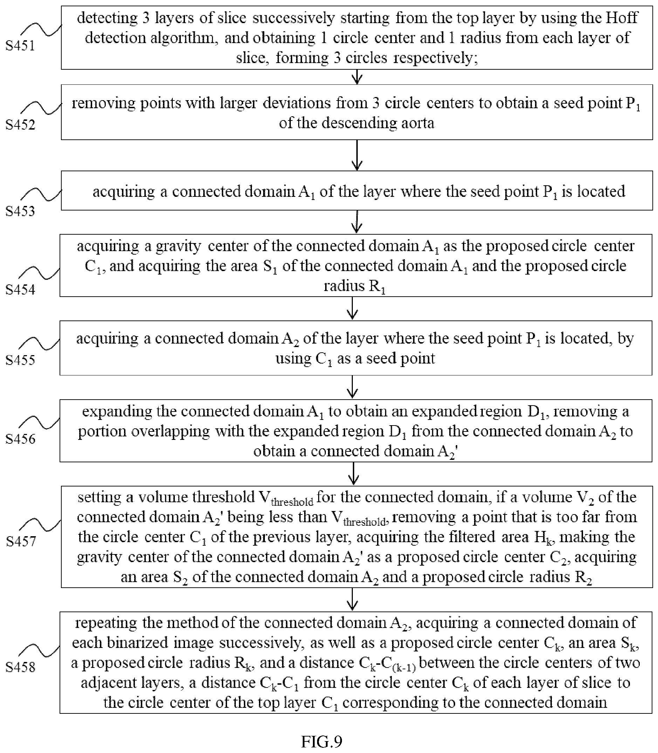

S450, as shown in FIG. 9, starting from the top layer, acquiring a connected domain of each binarized image successively, as well as a proposed circle center $C_k$, an area $S_k$, a proposed circle radius $R_k$, a distance $C_k-C_{(k-1)}$ between the circle centers of two adjacent layers, a distance $C_k-C_1$ from the circle center $C_k$ of each layer of slice to the circle center of the top layer $C_1$, and an area $M_k$ of all pixels whose pixel points are greater than 0 in a layer pixel and whose pixel points are equal to 0 in the previous layer pixel and a filtered area $H_k$ corresponding to the connected domain, where k denotes the k-th layer of slice and k≥ 1; the proposed circle center $C_k$, the distance $C_k-C_{(k-1)}$, the areas $S_k$, $M_k$, $H_k$, the proposed circle radius $R_k$, and the distance $L_{k-(k-1)}$ between two adjacent layers corresponding to the connected domain as feature data, comprising:

S451, detecting 3 layers of slice successively starting from the top layer by using the Hoff detection algorithm, and obtaining 1 circle center and 1 radius from each layer of slice, forming 3 circles respectively;

S452, removing points with larger deviations from 3 circle centers to obtain a seed point $P_1$ of the descending aorta;

S453, acquiring a connected domain $A_1$ of the layer where the seed point $P_1$ is located;

S454, acquiring a gravity center of the connected domain $A_1$ as the proposed circle center $C_1$, and acquiring the area $S_1$ of the connected domain $A_1$ and the proposed circle radius $R_1$;

S455, acquiring a connected domain $A_2$ of the layer where the seed point $P_1$ is located, by using $C_1$ as a seed point;

S456, expanding the connected domain $A_1$ to obtain an expanded region $D_1$, removing a portion overlapping with the expanded region $D_1$ from the connected domain $A_2$ to obtain a connected domain $A_2'$;

S457, setting a volume threshold $V_{threshold}$ for the connected domain, if a volume $V_2$ of the connected domain $A_2'$ being less than $V_{threshold}$, removing a point that is too far from the circle center $C_1$ of the previous layer, acquiring the filtered area $H_k$, making the gravity center of the connected domain $A_2'$ as a proposed circle center $C_2$, acquiring an area $S_2$ of the connected domain $A_2$ and a proposed circle radius $R_2$;

S458, repeating the method of the connected domain $A_2$, acquiring a connected domain of each binarized image successively, as well as a proposed circle center $C_k$, an area $S_k$, a proposed circle radius $R_k$, and a distance $C_k-C_{(k-1)}$ between the circle centers of two adjacent layers, a distance $C_k-C_1$ from the circle center $C_k$ of each layer of slice to the circle center of the top layer $C_1$ corresponding to the connected domain.

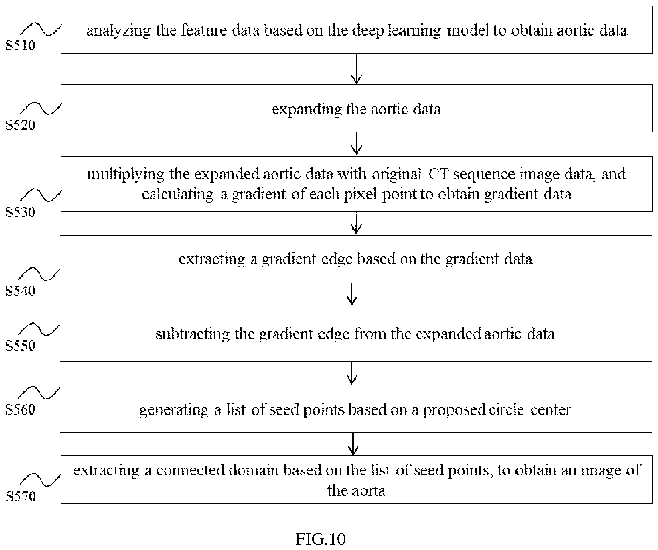

S500, as shown in FIG. 10, acquiring an image of the aorta from the CT sequence images based on the deep learning model and feature data, comprising:

S510, analyzing the feature data based on the deep learning model to obtain aortic data;

S520, expanding the aortic data;

S530, multiplying the expanded aortic data with original CT sequence image data, and calculating a gradient of each pixel point to obtain gradient data;

S540, extracting a gradient edge based on the gradient data;

S550, subtracting the gradient edge from the expanded aortic data;

S560, generating a list of seed points based on a proposed circle center;

S570, extracting a connected domain based on the list of seed points, to obtain an image of the aorta.

The present application provides a computer storage medium where a computer program is executed by a processor to implement the above method for acquiring an aorta based on deep learning.

Those skilled in the art know that aspects of the present invention can be implemented as systems, methods, or computer program products. As such, aspects of the present invention may be implemented in the form of: a fully hardware implementation, a fully software implementation (including firmware, resident software, microcode, etc.), or a combination of hardware and software aspects, collectively referred to herein as a "circuit", "module" or "system". In addition, in some embodiments, aspects of the present invention may also be implemented in the form of a computer program product in one or more computer-readable media containing computer-readable program code. Embodiments of the methods and/or systems of the present invention may be implemented in a manner that involves performing or completing selected tasks manually, automatically, or in a combination thereof.

For example, the hardware for performing the selected tasks based on the embodiments of the present invention may be implemented as a chip or circuit. As software, the selected tasks based on the embodiments of the present invention may be implemented as a plurality of software instructions to be executed by a computer using any appropriate operating system. In exemplary embodiments of the present invention, one or more tasks, as in the exemplary embodiments based on the methods and/or systems herein, is performed by a data processor, such as a computing platform for executing a plurality of instructions. Optionally, the data processor includes volatile storage for storing instructions and/or data, and/or non-volatile storage for storing instructions and/or data, such as a magnetic hard disk and/or removable media. Optionally, a network connection is also provided. Optionally, a display and/or user input device, such as a keyboard or mouse, is also provided.

Any combination of one or more computer readable may be utilized. A computer-readable medium may be a computer-readable signal medium or a computer-readable storage medium. A computer-readable storage medium may be, for example - but not limited to - an electrical, magnetic, optical, electromagnetic, infrared, or semiconductor system, device or component, or any combination thereof. More specific examples of computer-readable storage media (a non-exhaustive list) would include each of the following:

An electrical connection having one or more wires, a portable computer disk, a hard disk, a random access memory (RAM), a read-only memory (ROM), an erasable programmable read-only memory (EPROM or flash memory), an optical fiber, a portable compact disk read-only memory (CD-ROM), an optical storage component, a magnetic storage component, or any suitable combination of the foregoing. In this specification, the computer-readable storage medium may be any tangible medium that contains or stores a program that can be used by or in combination with an instruction execution system, device or component.

The computer-readable signal medium may include a data signal propagated in a baseband or as part of a carrier wave that carries computer-readable program code. This propagated data signal can take a variety of forms, including but not limited to electromagnetic signals, optical signals or any suitable combination of the above. The computer-readable signal medium may also be any computer-readable medium other than a computer-readable storage medium that sends, propagates, or transmits a program for being used by or in conjunction with an instruction execution system, device or component.

The program code contained on the computer-readable medium may be transmitted using any suitable medium, including (but not limited to) wireless, wired, fiber optic, RF, etc., or any suitable combination of the above.

For example, computer program code for performing operations of aspects of the present invention may be written in any combination of one or more programming languages, including object-oriented programming languages such as Java, Smalltalk, C++, and conventional procedural programming languages such as "C" programming language or the like. The program code may be executed entirely on an user's computer, partially on an user's computer, as a stand-alone software package, partially on an user's computer and partially on a remote computer, or entirely on a remote computer or server. In the case of a remote computer, the remote computer may be connected to an user's computer via any kind of network - including a local area network (LAN) or a wide area network (WAN) - or, may be connected to an external computer (e.g., using an Internet service provider to connect via the Internet).

It should be understood that each block of the flowchart and/or block diagram, and a combination of respective blocks in the flowchart and/or block diagram, may be implemented by computer program instructions. These computer program instructions may be provided to a processor of a general purpose computer, a specialized computer, or other programmable data processing device, thereby producing a machine such that these computer program instructions, when executed by the processor of the computer or other programmable data processing device, produce a device that implements a function/action specified in one or more of the blocks in the flowchart and/or block diagram.

These computer program instructions may also be stored in a computer-readable medium that causes a computer, other programmable data processing device, or other apparatus to operate in a particular manner such that the instructions stored in the computer-readable medium result in an article of manufacture that includes instructions to implement the function/action specified in one or more blocks in the flowchart and/or block diagram.

Computer program instructions may also be loaded onto a computer (e.g., a coronary artery analysis system) or other programmable data processing apparatus to cause a series of operational steps to be performed on the computer, other programmable data processing apparatus or other apparatus to produce a computer-implemented process, such that the instructions executed on the computer, other programmable device or other apparatus provide a process for implementing the function/action specified in a block of the flowchart and/or one or more block diagram.

The above specific examples of the present invention further detail the purpose, technical solutions and beneficial effects of the present invention. It should be understood that the above are only specific embodiments of the present invention and are not intended to limit the present invention, and that any modifications, equivalent replacements, improvements, etc. made within the spirit and principles of the present invention shall be included within the protection scope of the present invention.

**Claims**

1. A method for acquiring aorta based on deep learning, **characterized by** comprising:

    acquiring a database of slices of an aorticlayer and a database of slices of a non-aortic layer;
    performing deep learning on the database of slices of the aortic layer and the database of slices of the non-aortic layer, to obtain a deep learning model;
    acquiring CT sequence images to be processed or three-dimensional data of CT sequence images to be processed;
    extracting feature data from the CT sequence images to be processed or the three-dimensional data of the CT sequence images to be processed;
    acquiring an image of the aorta from the CT sequence images based on the deep learning model and the feature data.

2. The method for acquiring aorta based on deep learning according to claim 1, **characterized in that**, the manner for acquiring a database of slices of an aortic layer and a database of slices of a non-aortic layer comprises:

    removing new images of the lung, descending aorta, spine and ribs from CT sequence images;
    slicing starting from a top layer of the new images to obtain a group of two-dimensional images;
    binarizing the group of two-dimensional images to obtain a group of binarized images;
    generating an image of the aorta based on each group of binarized images;
    generating slice data of the aortic layer based on the image of the aorta, with the remaining slice data being as slice data of the non-aortic layer, and obtaining a database of slices of the aortic layer and a database of slices of the non-aortic layer.

3. The method for acquiring aorta based on deep learning according to claim 2, **characterized in that**, the manner for binarizing the group of two-dimensional images to obtain a group of binarized images comprises:

    setting a coronary tree grayscale threshold $Q_{coronary\ 1}$; based on
    $$\left\{ \begin{array}{l} 0 \leq Q_m < Q_{coronar\ y\ 1}, P(m) = 0 \\ Q_{coronary\ 1} \leq Q_m \leq 255, P(m) = 1 \end{array} \right\}_{,}$$ binarizing the slices of each layer of the new images with the lung, descending aorta, spine, and ribs removed to remove impurity points in the new images and obtain a group of binarized images;
    where m is a positive integer, $Q_m$ denotes the grayscale value corresponding to the m-th pixel point PO, and P(m) denotes the pixel value corresponding to the m-th pixel point PO.

4. The method for acquiring aorta based on deep learning according to claim 2, **characterized in that**, the manner for generating an image of the aorta based on each group of binarized images comprises:

    creating a search engine list for each layer of slices in the group of binarized images;
    searching for circles in each layer of slices, comparing the number of pixel points in the search engine list of each layer and the radii of the circles, and finding an eligible circle center point;
    searching for a circle center point of the next layer of slice, if no eligible circle center point can be found.

5. The method for acquiring aorta based on deep learning according to claim 1, **characterized in that**, the manner for removing new images of the lung, descending aorta, spine and ribs from CT sequence images comprises:

acquiring a three-dimensional database of CT sequence images;

plotting a grayscale histogram of each group of the CT sequence images;

along a direction of the end point M to the original point O of the grayscale histogram, acquiring a volume of each grayscale value region from point M to point M-1, from point M to point M-2, until from point M to point O; acquiring a volume ratio V of the volume of each grayscale value region to a volume of the total region from point M to point O;

setting a lung grayscale threshold $Q_{lung}$ based on medical knowledge and CT imaging principle; if a grayscale value in the grayscale histogram being less than $Q_{lung}$, removing an image corresponding to the grayscale value to obtain a first image with the lung tissue removed;

acquiring a gravity center of heart and a gravity center of spine corresponding to each group of CT sequence images based on the first image;

acquiring an image of descending aorta for each group of CT sequence images based on the gravity center of heart and the gravity center of spine;

removing the lung, descending aorta, spine, and ribs from the CT sequence images to acquire new images.

6. The method for acquiring aorta based on deep learning according to claim 5, **characterized in that**, the manner for acquiring a gravity center of heart and a gravity center of spine corresponding to each group of CT sequence images based on the first image comprises:

if V = b, picking a start point corresponding to the grayscale value region, projecting the start point onto the CT three-dimensional image, acquiring a three-dimensional image of a heart region, and picking a physical gravity center of the three-dimensional image of the heart region, which is the gravity center of heart $P_2$; wherein b denotes a constant, 0.2 < b < 1;

if V = a, picking a start point corresponding to the grayscale value region, projecting the start point onto the CT three-dimensional image, acquiring a three-dimensional image of a bone region, and picking a physical gravity center of the three-dimensional image of the bone region, which is the gravity center of spine $P_1$; wherein a denotes a constant, 0 < a < 0.2.

7. The method for acquiring aorta based on deep learning according to claim 6, **characterized in that**, the manner for acquiring an image of descending aorta for each group of CT sequence images based on the gravity center of heart and the gravity center of spine comprises:

setting a lung grayscale threshold $Q_{lung}$ based on medical knowledge and CT imaging principle;

if a grayscale value in the grayscale histogram being less than $Q_{lung}$, removing an image corresponding to the grayscale value to obtain a first image with the lung tissue removed;

projecting the gravity center of heart $P_2$ onto the first image to obtain a circle center of the heart $O_1$;

setting a grayscale threshold for the descending aorta $Q_{descending}$, and binarizing the first image;

acquiring a circle corresponding to the descending aorta based on a distance from the descending aorta to the circle center of the heart $O_1$ and a distance from the spine to the circle center of the heart $O_1$;

acquiring an image of the descending aorta from the CT sequence image.

8. The method for acquiring aorta based on deep learning according to claim 7, **characterized in that**, the manner for setting a grayscale threshold for the descending aorta $Q_{descending}$ and binarizing the first image comprises:

acquiring one or more pixel points PO within the first image with a grayscale value greater than the grayscale threshold for the descending aorta $Q_{descending}$, and calculating an average grayscale value $\overline{Q}_1$ of the one or more pixel points PO;

layered slicing the first image starting from its bottom layer to obtain a first group of two-dimensional sliced images;

based on

$$\left\{ \begin{array}{l} Q_k < Q_{descending} \ , P(k) = 0 \\ Q_{descending} \ \leq Q_k \leq 2\overline{Q}_1, \ P(k) = 1 \\ Q_k > 2\overline{Q}_1, P(k) = 0 \end{array} \right\},$$

binarizing the first image, removing impurity points in the first image to obtain a binarized image, wherein k is a positive integer, $Q_k$ denotes the grayscale value corresponding to the k-th pixel point PO, and P(k) denotes the pixel value corresponding to the k-th pixel point PO.

9. The method for acquiring aorta based on deep learning according to claim 8, **characterized in that**, the manner for acquiring a circle corresponding to the descending aorta based on a distance from the descending aorta to the circle center of the heart $O_1$ and a distance from the spine to the circle center of the heart $O_1$ comprises:

setting an radius threshold of the circle formed from the descending aorta to an edge of the heart to $r_{threshold}$;

acquiring an approximate region of the spine and an approximate region of the descending aorta based on the distance between the descending aorta and the heart being less than the distance between the spine and the heart;

removing one or more error pixel points based on the approximate region of the descending aorta, and obtaining an image of the descending aorta, i.e., a circle corresponding to the descending aorta.

10. The method for acquiring aorta based on deep learning according to claim 9, **characterized in that**, the manner for acquiring an approximate region of the spine and an approximate region of the descending aorta based on the distance between the descending aorta and the heart being less than the distance between the spine and the heart comprises that:

if a circle obtained by the Hoff detection algorithm meets the condition that its radius r > $r_{threshold}$, then this circle is the circle corresponding to the spine and is the approximate region of the spine, and the center and radius need not to be recorded;

if a circle obtained by the Hoff detection algorithm meets the condition that its radius r <- $r_{threshold}$, then this circle may be the circle corresponding to the descending aorta and is the approximate region of the descending aorta, and the center and radius need to be recorded.

11. The method for acquiring aorta based on deep learning according to claim 10, **characterized in that**, the manner for extracting feature data from the CT sequence images to be processed or the three-dimensional data of the CT sequence images to be processed comprises:

plotting a grayscale histogram of the CT sequence images to be processed;

acquiring an average grayscale value $\overline{Q}_1$ of one or more pixel points with a grayscale value greater than $Q_{descending}$;

layered slicing the CT sequence images to obtain a group of two-dimensional sliced images;

binarizing all the images in the group of two-dimensional sliced images based on the average grayscale value $\overline{Q}_1$, to obtain a plurality of binarized images;

acquiring a connected domain of each binarized image successively starting from the top layer, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer $C_1$ corresponding to the connected domain, and an area $M_k$ of all pixels whose pixel points are greater than 0 in a layer pixel and whose pixel points are equal to 0 in the previous layer pixel and a filtered area $H_k$, wherein k denotes the k-th layer of slice, $k \geq 1$;

making the proposed circle center $C_k$, the distance $C_k$-$C_{(k-1)}$, the areas $S_k$, $M_k$, $H_k$, the proposed circle radius $R_k$, and the distance $L_{k-(k-1)}$ between two adjacent layers corresponding to the connected domain as feature data.

12. The method for acquiring aorta based on deep learning according to claim 11, **characterized in that**, the manner for acquiring an image of the aorta from the CT sequence images based on the deep learning model and the feature data comprises:

analyzing the feature data based on the deep learning model to obtain aortic data;

expanding the aortic data;

multiplying the expanded aortic data with original CT sequence image data, and calculating a gradient of each pixel point to obtain gradient data;

extracting a gradient edge based on the gradient data;

subtracting the gradient edge from the expanded aortic data;

generating a list of seed points based on a proposed circle center;

extracting a connected domain based on the list of seed points, to obtain an image of the aorta.

13. The method for acquiring aorta based on deep learning according to claim 12, **characterized in that**, the manner for acquiring a connected domain of each binarized image successively starting from the top layer, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer $C_1$ corresponding to the connected domain, and an area $M_k$ of all pixels whose pixel points are greater than 0 in a layer pixel and whose pixel points are equal to 0 in the previous layer pixel and a filtered area $H_k$, wherein k denotes the k-th layer of slice, $k \geq 1$, comprises:

detecting 3 layers of slice successively starting from the top layer by using the Hoff detection algorithm, and obtaining 1 circle center and 1 radius from each layer of slice, forming 3 circles respectively;

removing points with larger deviations from 3 circle centers to obtain a seed point $P_1$ of the descending aorta;

acquiring a connected domain $A_1$ of the layer where the seed point $P_1$ is located;

acquiring a gravity center of the connected domain $A_1$ as the proposed circle center $C_1$, and acquiring the area $S_1$ of the connected domain $A_1$ and the proposed circle radius $R_1$;

acquiring a connected domain $A_2$ of the layer where the seed point $P_1$ is located, by using the $C_1$ as a seed point;

expanding the connected domain $A_1$ to obtain an expanded region $D_1$, removing a portion overlapping with the expanded region $D_1$ from the connected domain $A_2$ to obtain a connected domain $A_2$';

setting a volume threshold $V_{threshold}$ for the connected domain, if a volume $V_2$ of the connected domain $A_2$' being less than $V_{threshold}$, removing a point that is too far from the circle center $C_1$ of the previous layer, acquiring the filtered area $H_k$, making the gravity center of the connected domain $A_2$' as a proposed circle center $C_2$, acquiring an area $S_2$ of the connected domain $A_2$ and a proposed circle radius $R_2$;

repeating the method of the connected domain $A_2$, acquiring a connected domain of each binarized image successively, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer $C_1$ corresponding to the connected domain.

**14.** A computer storage medium, **characterized in that**, the method for acquiring aorta based on deep learning according to any one of claims 1 to 13 is implemented when a computer program is executed by a processor.

S100 — acquiring a database of slices of an aortic layer and a database of slices of a non-aortic layer

S200 — performing deep learning on the database of slices of the aortic layer and the database of slices of the non-aortic layer, to obtain a deep learning model

S300 — acquiring CT sequence images to be processed or three-dimensional data of CT sequence images to be processed

S400 — extracting feature data from the CT sequence images to be processed or the three-dimensional data of the CT sequence images to be processed

S500 — acquiring an image of the aorta from the CT sequence images based on the deep learning model and the feature data

FIG.1

S110 — removing new images of the lung, descending aorta, spine and ribs from CT sequence images

S120 — slicing starting from a top layer of the new images to obtain a group of two-dimensional images

S130 — binarizing the group of two-dimensional images to obtain a group of binarized images

S140 — generating an image of the aorta based on each group of binarized images

S150 — generating slice data of the aortic layer based on the image of the aorta, with the remaining slice data being as slice data of the non-aortic layer, and obtaining a database of slices of the aortic layer and a database of slices of the non-aortic layer

FIG.2

S111 — acquiring a three-dimensional database of CT sequence images

↓

S112 — plotting a grayscale histogram of each group of CT sequence images

↓

S113 — along a direction of the end point M to the original point O of the grayscale histogram, acquiring a volume of each grayscale value region from point M to point M-1, from point M to point M-2, until from point M to point O; acquiring a volume ratio V of the volume of each grayscale value region to a volume of the total region from point M to point O

↓

S114 — setting a lung grayscale threshold $Q_{lung}$ based on medical knowledge and CT imaging principle; if a grayscale value in the grayscale histogram being less than $Q_{lung}$, removing an image corresponding to the grayscale value to obtain a first image with the lung tissue removed

S115 — acquiring a gravity center of heart and a gravity center of spine corresponding to each group of CT sequence images based on the first image

↓

S116 — acquiring an image of descending aorta for each group of CT sequence images based on the gravity center of heart and the gravity center of spine

↓

S117 — removing the lung, descending aorta, spine and ribs from the CT sequence images to acquire new images

FIG.3

S1161 — setting a lung grayscale threshold $Q_{lung}$ based on medical knowledge and CT imaging principle

S1162 — if a grayscale value in the grayscale histogram being less than $Q_{lung}$, removing an image corresponding to the grayscale value to obtain a first image with the lung tissue removed

S1163 — projecting the gravity center of heart $P_2$ onto the first image to obtain a circle center of the heart $O_1$

S1164 — setting a grayscale threshold for the descending aorta $Q_{descending}$, and binarizing the first image

S1165 — acquiring a circle corresponding to the descending aorta based on a distance from the descending aorta to the circle center of the heart $O_1$ and a distance from the spine to the circle center of the heart $O_1$; acquiring an image of the descending aorta from the CT sequence images

FIG.4

S11641 — acquiring one or more pixel points PO within the first image with a grayscale value greater than the grayscale threshold for the descending aorta $Q_{descending}$, and calculating an average grayscale value $\overline{Q}_1$ of the one or more pixel points PO

S11642 — layered slicing the first image starting from its bottom layer to obtain a first group of two-dimensional sliced images

S11643 — binarizing a sliced image of the first image, removing impurity points in the first image to obtain a binarized image

FIG.5

| S11651 | setting an radius threshold of the circle formed from the descending aorta to an edge of the heart to $r_{threshold}$ |
|---|---|

| S11652 | acquiring an approximate region of the spine and an approximate region of the descending aorta based on the distance between the descending aorta and the heart being less than the distance between the spine and the heart |
|---|---|

| S11653 | removing one or more error pixel points based on the approximate region of the descending aorta, and obtaining a circle corresponding to the descending aorta |
|---|---|

FIG.6

| S141 | creating a search engine list for each layer of slices in the group of binarized images |
|---|---|

| S142 | searching for circles in each layer of slices, comparing the number of pixel points in the search engine list of each layer and the radii of the circles, and finding an eligible circle center point |
|---|---|

| S143 | searching for a circle center point of the next layer of slice, if no eligible circle center can be found |
|---|---|

FIG.7

S410 — plotting a grayscale histogram of the CT sequence images to be processed

S420 — acquiring an average grayscale value of one or more pixel points with a grayscale value greater than $Q_{descending}$

S430 — layered slicing the CT sequence images to obtain a group of two-dimensional sliced images

S440 — binarizing all the images in the group of two-dimensional sliced images based on the average grayscale value, to obtain a plurality of binarized images

S450 — acquiring a connected domain of each binarized image successively, as well as a proposed circle center $C_k$, an area $S_k$, a proposed circle radius $R_k$, a distance $C_k$-$C_{(k-1)}$ between the circle centers of two adjacent layers, a distance $C_k$-$C_1$ from the circle center $C_k$ of each layer of slice to the circle center of the top layer $C_1$, and an area $M_k$ of all pixels whose pixel points are greater than 0 in a layer pixel and whose pixel points are equal to 0 in the previous layer pixel and a filtered area $H_k$ corresponding to the connected domain, where k denotes the k-th layer of slice and $k \geq 1$; the proposed circle center $C_k$, the distance $C_k$-$C_{(k-1)}$, the areas $S_k$, $M_k$, $H_k$, the proposed circle radius $R_k$, and the distance $L_{k-(k-1)}$ between two adjacent layers corresponding to the connected domain as feature data

FIG.8

S451 — detecting 3 layers of slice successively starting from the top layer by using the Hoff detection algorithm, and obtaining 1 circle center and 1 radius from each layer of slice, forming 3 circles respectively;

$\downarrow$

S452 — removing points with larger deviations from 3 circle centers to obtain a seed point $P_1$ of the descending aorta

$\downarrow$

S453 — acquiring a connected domain $A_1$ of the layer where the seed point $P_1$ is located

$\downarrow$

S454 — acquiring a gravity center of the connected domain $A_1$ as the proposed circle center $C_1$, and acquiring the area $S_1$ of the connected domain $A_1$ and the proposed circle radius $R_1$

$\downarrow$

S455 — acquiring a connected domain $A_2$ of the layer where the seed point $P_1$ is located, by using $C_1$ as a seed point

$\downarrow$

S456 — expanding the connected domain $A_1$ to obtain an expanded region $D_1$, removing a portion overlapping with the expanded region $D_1$ from the connected domain $A_2$ to obtain a connected domain $A_2'$

$\downarrow$

S457 — setting a volume threshold $V_{threshold}$ for the connected domain, if a volume $V_2$ of the connected domain $A_2'$ being less than $V_{threshold}$, removing a point that is too far from the circle center $C_1$ of the previous layer, acquiring the filtered area $H_k$, making the gravity center of the connected domain $A_2'$ as a proposed circle center $C_2$, acquiring an area $S_2$ of the connected domain $A_2$ and a proposed circle radius $R_2$

$\downarrow$

S458 — repeating the method of the connected domain $A_2$, acquiring a connected domain of each binarized image successively, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer $C_1$ corresponding to the connected domain

FIG.9

| | |
|---|---|
| S510 | analyzing the feature data based on the deep learning model to obtain aortic data |

↓

| | |
|---|---|
| S520 | expanding the aortic data |

↓

| | |
|---|---|
| S530 | multiplying the expanded aortic data with original CT sequence image data, and calculating a gradient of each pixel point to obtain gradient data |

↓

| | |
|---|---|
| S540 | extracting a gradient edge based on the gradient data |

↓

| | |
|---|---|
| S550 | subtracting the gradient edge from the expanded aortic data |

↓

| | |
|---|---|
| S560 | generating a list of seed points based on a proposed circle center |

↓

| | |
|---|---|
| S570 | extracting a connected domain based on the list of seed points, to obtain an image of the aorta |

FIG.10

## INTERNATIONAL SEARCH REPORT

| | |
|---|---|
| International application No. | |
| | **PCT/CN2020/132796** |

**A. CLASSIFICATION OF SUBJECT MATTER**

G06T 7/00(2017.01)i; G06N 20/00(2019.01)i

According to International Patent Classification (IPC) or to both national classification and IPC

**B. FIELDS SEARCHED**

Minimum documentation searched (classification system followed by classification symbols)

G06T G06N

Documentation searched other than minimum documentation to the extent that such documents are included in the fields searched

Electronic data base consulted during the international search (name of data base and, where practicable, search terms used)

CNPAT, CNKI, WPI, EPODOC, IEEE: 主动脉, 切片, 横截面, 深度学习, 机器学习, 神经网络, 三维, CT, Computed Tomographic, aorta, deep learning, neural network, cross-sectional, slice, 3D, three dimensional

**C. DOCUMENTS CONSIDERED TO BE RELEVANT**

| Category* | Citation of document, with indication, where appropriate, of the relevant passages | Relevant to claim No. |
|---|---|---|
| X | CN 109035255 A (SOUTHEAST UNIVERSITY) 18 December 2018 (2018-12-18) claims 1-3, description paragraphs [0034]-[0058] | 1, 14 |
| Y | CN 109035255 A (SOUTHEAST UNIVERSITY) 18 December 2018 (2018-12-18) claims 1-3, description, paragraphs [0034]-[0058] | 2-10 |
| PY | CN 111815583 A (SUZHOU RUNXIN MEDICAL INSTRUMENT CO., LTD.) 23 October 2020 (2020-10-23) description paragraphs [0006]-[0076] | 2-10 |
| PY | CN 111815587 A (SUZHOU RUNXIN MEDICAL INSTRUMENT CO., LTD.) 23 October 2020 (2020-10-23) description, paragraphs [0060]-[0077] | 2-4 |
| PY | CN 111815588 A (SUZHOU RUNXIN MEDICAL INSTRUMENT CO., LTD.) 23 October 2020 (2020-10-23) description paragraphs [0075]-[0101] | 5-10 |
| A | CN 110264465 A (CENTRAL SOUTH UNIVERSITY OF FORESTRY AND TECHNOLOGY) 20 September 2019 (2019-09-20) entire document | 1-14 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **10 March 2021** | **30 March 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**EP 4 174 760 A1**

<table>
<tr><td colspan="3" align="center">INTERNATIONAL SEARCH REPORT</td><td colspan="2">International application No.<br>**PCT/CN2020/132796**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | CN 106803251 A (XIDIAN UNIVERSITY) 06 June 2017 (2017-06-06)<br>entire document | 1-14 |
| A | US 2019019287 A1 (SIEMENS HEALTHCARE GMBH) 17 January 2019 (2019-01-17)<br>entire document | 1-14 |

Form PCT/ISA/210 (second sheet) (January 2015)

22

**INTERNATIONAL SEARCH REPORT**
Information on patent family members

International application No.

**PCT/CN2020/132796**

| Patent document cited in search report | | | Publication date (day/month/year) | Patent family member(s) | | | Publication date (day/month/year) |
|---|---|---|---|---|---|---|---|
| CN | 109035255 | A | 18 December 2018 | WO | 2020001217 | A1 | 02 January 2020 |
| CN | 111815583 | A | 23 October 2020 | | None | | |
| CN | 111815587 | A | 23 October 2020 | | None | | |
| CN | 111815588 | A | 23 October 2020 | | None | | |
| CN | 110264465 | A | 20 September 2019 | | None | | |
| CN | 106803251 | A | 06 June 2017 | | None | | |
| US | 2019019287 | A1 | 17 January 2019 | EP | 3432215 | A1 | 23 January 2019 |

Form PCT/ISA/210 (patent family annex) (January 2015)